# FASCICULE DE BREVET EUROPEEN

(11) **EP 0 584 477 B1**
(45) Date de publication et mention de la délivrance du brevet: **24.09.1997**
(21) Numéro de dépôt: 93109656.4
(22) Date de dépôt: 17.06.1993
(51) Int. Cl.: C11B 9/00, A61K 7/46

(54) **Utilisation d'une cyclopentadécénone à titre d'ingrédient parfumant**
Verwendung einer Cyclopentadecenone als Parfümszutat
Use of a cyclopentadecenone as a perfuming ingredient

(30) Priorité: 30.07.1992 CH 2395/92
(43) Date de publication de la demande: 02.03.1994
(73) Titulaire: FIRMENICH SA, CH-1211 Genève 8 (CH)
(72) Inventeur: Demole, Edouard, Dr., CH-1296 Coppet (CH); Mahaim, Cyril, Dr., CH-1112 Echichens (CH); Blanc, Pierre-Alain, Dr., CH-1263 Crassier (CH)
(74) Mandataire: Salvaterra-Garcia, Maria de Lurdes

(56) Documents cités:
- EP-A- 0 015 412
- EP-A- 0 025 869
- FR-A- 1 558 413
- FR-A- 2 464 936
- S.ARCTANDER 'Perfume and Flavor Chemicals' 1969 , S.ARCTANDER , MONTCLAIR,N.J. *numeros:813,1982-1985,2276*

## Description

La présente invention a trait au domaine de la parfumerie. Elle concerne plus particulièrement l'utilisation de la 3-méthyl-cyclopentadéc-5-én-1-one à titre d'ingrédient parfumant.

La 3-méthyl-cyclopentadéc-5-én-1-one est une cétone macrocyclique et un homologue insaturé de la muscone, un composé fort apprécié en parfumerie. Compte tenu de la présence d'une double liaison cyclique dans sa molécule, nombreux sont les isomères de position et de configuration possible de cette cétone. Certains sont de structure connue. C'est ainsi que la structure de l'énone susmentionnée, ainsi que celles de plusieurs de ses isomères, ont été décrites dans la littérature, pour la plupart dans le cadre de synthèses organiques, notamment celle de la muscone, mais sans qu'il y ait mention d'éventuelles propriétés olfactives intéressantes de ces composés.

Une exception, cependant, est le brevet US 3,778,483 qui décrit un procédé de préparation de la muscone à partir de cétones insaturées de formule 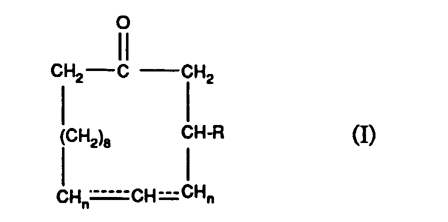 possédant une double liaison de configuration cis ou trans dans l'une des positions indiquées par les pointillés et dans laquelle R représente l'hydrogène ou un radical méthyle, l'un des indices n a la valeur 1 et l'autre la valeur 2. Les cétones de formule (I) sont dites posséder une odeur plus forte que celle de leurs cétones homologues saturées correspondantes. Selon les inventeurs du brevet cité, les cétones saturées posséderaient une odeur musquée caractéristique, tandis que les cétones (I) où R est l'hydrogène posséderaient une odeur qui rappelle celle de la civettone et celles où R est méthyle une odeur plutôt boisée. En dépit du fait que plusieurs cétones (I) ou mélanges de cétones (I) sont cités dans ce brevet, notamment les 3-méthyl-cis-cyclopentadéc-4-én-1-one (exemple 4) et 3-méthyl-trans-cyclopentadéc-4-én-1-one (exemple 17), ainsi qu'un mélange contenant essentiellement les isomères trans de la 3-méthyl-cyclopentadéc-4 et 5-én-1-one (exemples 1 et 2), nous n'avons pu trouver aucune description des propriétés individuelles de ces cétones ou mélanges, ni des éventuelles différences olfactives entre elles.

D'autre part, un brevet plus récent, à savoir GB 2 058 786, fait état de la préparation de cétones macrocycliques insaturées de formule 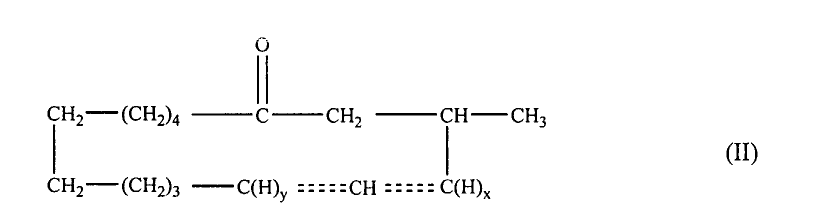 dans laquelle les lignes pointillées désignent l'emplacement d'une liaison simple ou double et dans laquelle x=1 et y=2, ou x=2 et y=1. Bien que l'on cite l'intérêt des propriétés olfactives des cétones (II) (voir page 4, ligne 18), cette citation est basée sur l'art antérieur cité plus haut et aucune description plus détaillée des propriétés de la 3-méthyl-cyclopentadéc-4-én-1-one, décrite à l'exemple 5 et désignée sous le nom de "muscénone", ne peut être trouvée dans ce document.

On connait également de l'art antérieur le brevet EP-A-0 025 869, qui décrit la 3-méthyl-cyclohexadéc-5-én-1-one, énone qui est dite posséder une odeur musquée rappelant celle de la muscone.

Finalement, un texte de base en parfumerie, à savoir l'ouvrage de S. Arctander, Perfume and Flavor Chemicals, Montclair, N.J., USA (1969), décrit à la section 1985, la 3-méthyl cyclopentadécénone, également désignée comme "muscénone", à laquelle est attribuée la structure 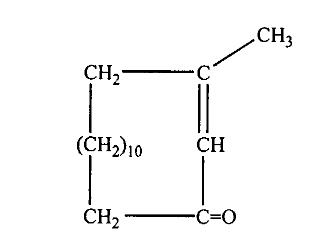 et dont l'odeur est dite être une "odeur musquée délicate mais extrêmement tenace développant, en dilution, des notes plus boisées, pas aussi moelleuses et veloutées que celles de la Muscone, peut-être comparables en intensité, mais inférieures en beauté" (sic). Curieusement, l'auteur affirme aussi "qu'il est inconcevable que cette cétone devienne un musc courant" (sic).

Alliées à une absence complète de caractérisation analytique des composés décrits, qui ne permet pas un enseignement précis de la nature des composés et mélanges obtenus selon l'art antérieur, ces descriptions olfactives peu détaillées et même quelque peu contradictoires, qui par ailleurs ne s'appuient pas sur des exemples d'utilisation en parfumerie, suggèrent simplement que toutes les cétones insaturées de formule (I) ou (II), homologues de la muscone et de l'Exaltone ® (cyclopentadécanone ; origine: Firmenich SA) possèdent des odeurs similaires, indépendemment de la position de la double liaison ou de la configuration de celle-ci.

Or, nous avons maintenant découvert avec surprise que tel n'est pas du tout le cas et que, si toutes les cétones de formule (II) sont en effet des substances odorantes, leurs propriétés olfactives sont distinctes l'une de l'autre, l'une de ces cétones possédant des propriétés particulièrement intéressantes et inattendues.

Nous avons en effet constaté que la 3-méthyl-cyclopentadéc-5-én-1-one possède une odeur bien musquée légèrement animale avec un caractère fortement nitré, en dépit de l'absence d'azote dans la molécule. Cette note olfactive est d'autant plus utile que l'on sait que l'emploi des composés nitrés à caractère musqué est de plus en plus limité pour des raisons d'innocuité et de protection de l'environnement. Au vu de l'art antérieur discuté plus haut le caractère et l'utilité de cette note apparaissent ainsi comme tout à fait inattendus.

Par ailleurs, nous avons également observé que les deux isomères de configuration de la 3-méthyl-cyclopentadéc-5-én-1-one possèdent des odeurs qui se distinguent l'une de l'autre, l'isomère as étant préféré pour sa note plus puissante, plus musquée et élégante mais moins animale que celle de l'isomère trans, cette dernière combinant le caractère musc-nitré avec une note graines d'ambrette plus marquée. Dans un cas, comme dans l'autre, il s'agit de notes odorantes qui se sont révélées très tenaces aussi bien sur mouillette que sur le linge, l'odeur de l'isomère cis étant cependant plus tenace que celle de l'isomère trans.

Il apparaît à l'évidence que si les propriétés originales et surprenantes de la 3-méthyl-cyclopentadéc-5-én-1-one, en particulier de son isomère cis, sont passées tout à fait inaperçues dans l'art antérieur, c'est que ces composés n'avaient pas été obtenus à l'état pur. En effet, ces composés ne sont pas spécifiquement décrits dans les brevets US 3,778,483 et GB 2 058 786, même si les formules générales indiquées les englobent. Le brevet US décrit soit des mélanges d'isomères, contenant aussi bien la 3-méthyl-cyclopentadéc-5-én-1-one que la 3-méthyl-cyclopentadéc-4-én-1-one, soit l'isomère cis de cette dernière, tandis que le brevet GB 2 058 786 ne décrit que la préparation de la 3-methyl-cyclopentadéc-4-én-1-one sans spécification des proportions isomériques. Or, nous avons découvert maintenant que cette dernière énone exhale une odeur où le caractère musqué est relativement faible, son isomère cis étant presque inodore, tandis que son isomère trans est doté d'une note musquée faible et sans caractère. Ces trois composés possèdent des odeurs totalement dépourvues du caractère musc-nitré qui rend la 3-méthyl-cyclopentadec-5-én-1-one et ses isomères aussi utiles. Cette remarquable différence d'odeurs entre les isomères de position 4 et 5 de la 3-méthylcyclopentadécénone montre une fois encore, si besoin était, le caractère imprévisible de toute découverte dans le domaine de la parfumerie, où une simple modification moléculaire peut entraîner des modifications parfois fondamentales dans la note olfactive d'un composé donné. Les documents de l'art antérieur auraient amené l'homme du métier à conclure que toutes les cétones insaturées homologues de la muscone et de l'Exaltone ® possèdent des odeurs similaires, plus boisées que musquées; certainement ils ne suggèrent pas que l'une de ces cétones en particulier, à savoir la 3-méthyl-cyclopentadéc-5-én-1-one, ainsi que ses isomères de configuration, possède des propriétés olfactives originales et surprenantes, rappelant les odeurs des muscs nitrés connus sous les noms de musc cétone et musc ambrette (voir S. Arctander, ouvrage cité, sects. 2279 et 2278 respectivement). Cette découverte est à la base de la présente invention.

De par leurs propriétés odorantes, la 3-méthyl-cyclopentadéc-5-én-1-one, et ses isomères cis et trans, se prêtent aussi bien pour des applications en parfumerie fine qu'en parfumerie fonctionelle. Leur tenacité sur linge les rend particulièrement utiles dans le parfumage de détergents et adoucissants textiles. Ils peuvent être employés seuls ou en mélange avec d'autres ingrédients parfumants, des solvants ou des adjuvants d'usage courant dans l'art. En particulier, nous avons pu établir que des mélanges de la 3-méthyl-cyclopentadéc-5-én-1-one avec la 3-méthyl-cyclopentadéc-4-én-1-one, contenant au moins 70% de la première, se sont révélés des ingrédients parfumants particulièrement avantageux selon l'invention, car, tout en préservant les caractéristiques olfactives désirées, de tels mélanges peuvent être obtenus de façon plus économique que la 3-méthyl-cyclopentadéc-5-én-1-one, ou ses isomères, à l'état pur. Ces mélanges sont nouveaux et font également l'objet de la présente invention. Des mélanges préférés contiennent au moins 30% en poids de 3-méthyl-cyclopentadéc-5-én-1-one sous forme de son isomère de configuration cis. Dans les exemples d'application suivants, lorsqu'on fait référence à la 3-méthyl-cyclopentadéc-5-én-1-one, on entend désigner aussi bien les isomères de configuration cis ou trans à l'état pur que tout mélange de ces isomères, ou encore les mélanges selon l'invention cités plus haut.

Les proportions dans lesquelles ces composés peuvent être utilisés dépendent de l'effet olfactif désiré, ainsi que de la nature des autres co-ingrédients parfumants. A titre d'exemple, on peut citer des concentrations de l'ordre de 5 à 10%, voire même 20% ou plus en poids, par rapport au poids de la composition parfumante ou du parfum dans lesquels ils sont incorporés.

Lors de leur utilisation pour le parfumage d'articles fonctionnels tels des savons, gels de douche ou bain, shampoings ou autres produits d'hygiène capillaire, préparations cosmétiques, désodorisants corporels ou d'air ambiant, détergents ou adoucissants textiles, ou produits d'entretien, des concentrations bien inférieures seront généralement employées.

Les composés et mélanges de l'invention ont été préparés comme suit:
Dans un ballon tricol avec pierre à ébullition, équipé d'un séparateur d'eau, d'un réfrigérant et d'un thermomètre, on a placé 1170,9 g de 14-méthyl-16-oxabicyclo[10.3.1]hexadéc-1(15)-ène (préparé comme il est décrit dans GB 2 058 786) et 2900 ml de toluène. On a chauffé à 130° pour refluer le toluène à travers le séparateur d'eau. Lorsque toute l'eau a été éliminée, on a ajouté 57,5 g d'acide phosphorique à 85%. On a laissé chauffer 5 1/2 h sous agitation vigoureuse. Le mélange réactionnel a été lavé à l'eau, puis au carbonate de sodium à 10%. On a concentré et distillé sur résidus pour obtenir 1013 g (rend. 86,5%) d'un mélange contenant 40,7% en poids de (E)-3-méthyl-cyclopentadéc-5-én-1-one, 31,2% en poids de (Z)-3-méthyl-cyclopentadéc-5-én-1-one, 13,8% en poids de (E)-3-méthyl-cyclopentadéc-4-én-1-one et 1,5% en poids de (Z)-3-méthyl-cyclopentadéc-4-én-1-one, ainsi que des quantités mineures d'autres isomères non-intéressants.
En soumettant ce mélange à des séparations chromatographiques répétées sur colonnes de silica gel et de silica gel/AgNO₃, en utilisant en tant qu'éluants du toluène ou de l'hexane contenant de 1 à 5% d'acétate d'éthyle, on a obtenu après distillation (P.éb. env. 100°C/6,7 Pa), les composés suivants :

### (E)-3-méthyl-cyclopentadéc-5-én-1-one (pure à 96%)

IR(Liq.) : 1710(C=O), 970(CH=CH, trans) cm⁻¹
RMN(¹H, 360MHz) : 0,93(d,J=5,7Hz,3H) δ ppm
RMN(¹³C) : 212(s, C=O) ; 132,7/129,6(2d, CH=CH) ; 49,2/41,5(2t, CH₂COCH₂); 39,8/31,5(2t, CH₂C=CCH₂) ; 28,4(d, CH) δ ppm
SM : 236(M⁺, 7), 178(7), 95(58), 82(62), 81(100), 69(45), 68(82), 67(81) 55(85), 47(75)

### (Z)-3-méthyl-cyclopentadéc-5-én-1-one

IR(Liq.) : 1710(C=O), env. 700 (CH=CH, cis) cm⁻¹
RMN(¹H, 360MHz) : 1,00(d,J=7,8Hz,3H) δ ppm
RMN(¹³C) : 211,6(s, C=O) ; 132,0/127,1(2d, CH=CH) ; 49,4/42,4(2t, CH₂COCH₂) ; 33,5/26,2(2t, CH₂C=CCH₂) ; 29,7(d, CH) δ ppm
SM : 236(M⁺, 8), 178(6), 95(43), 81(63), 79(40), 69(47), 68(87), 67(100), 55(79), 41(92)

L'invention sera maintenant décrite plus en détail à l'aide des exemples suivants:

### Exemple 1

### Préparation d'une composition parfumante

On a préparé une composition parfumante de base par mélange des ingrédients suivants:

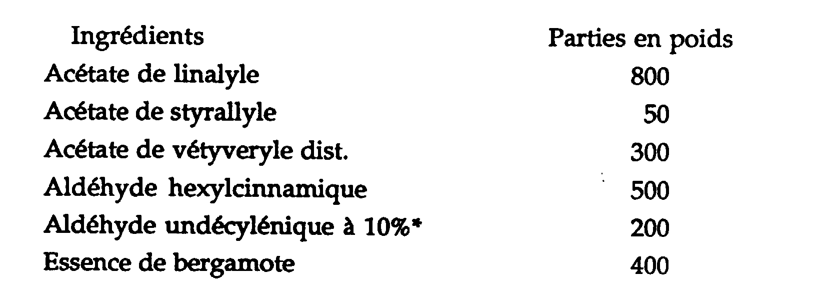

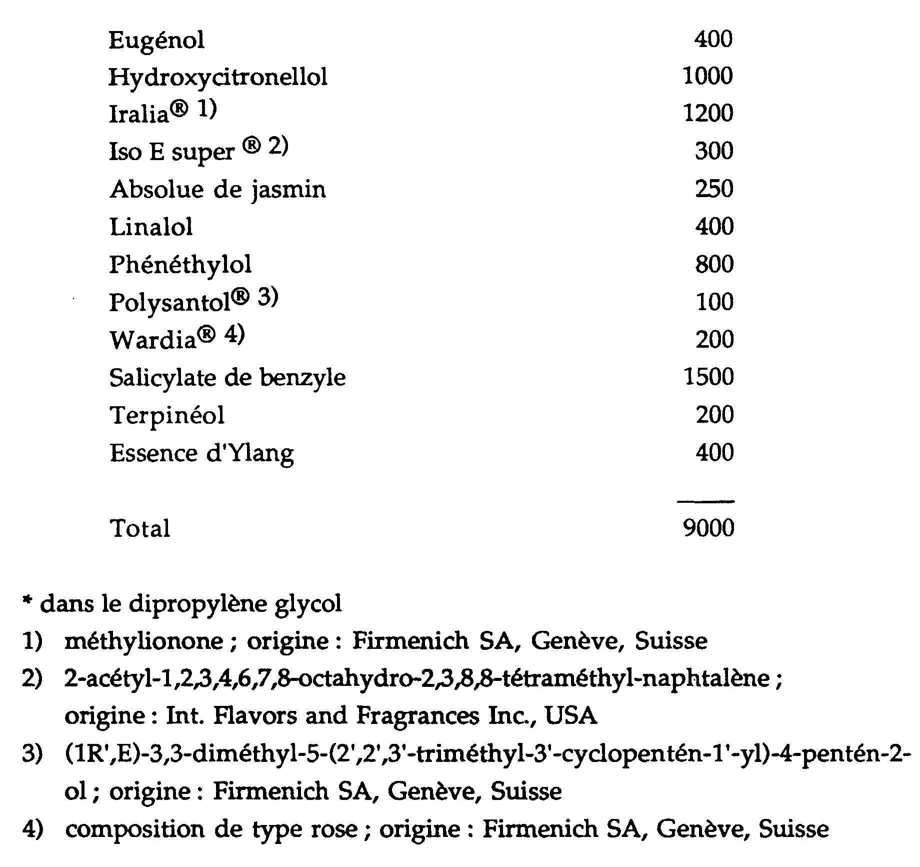

Lorsqu'on a ajouté à cette composition de base de type fleuri, épicé, 1000 parties en poids de 3-méthyl-cyclopentadéc-5-én-1-one, on a obtenu une nouvelle composition dont l'odeur avait acquis un net caractère musqué-doux, évoquant l'odeur de la musc cétone. L'effet olfactif était tout à fait semblable à celui que l'on a obtenu lorsqu'on a justement ajouté à cette composition de base 1000 parties en poids de ladite musc cétone, avec toutefois la différence que le composé de l'invention conférait un caractère plus fleuri à la composition.

En remplaçant la 3-méthyl-cyclopentadéc-5-én-1-one par la même quantité de l'un des produits du commerce de type musqué tels que l'Astrotone® (voir S. Arctander, ouvrage cité, sect. 1214), l'Exaltolide® (pentadécanolide ; origine: Firmenich SA, Genève, Suisse), la Galaxolide® (1,3,4,6,7,8-hexahydro-4,6,6,7,8,8-hexaméthyl-cyclopenta-γ-2-benzopyran; origine: International Flavors & Fragrances, USA), la muscone, la civettone (cycloheptadécénone ; origine : Firmenich SA, Genève, Suisse) ou la Tonalid ® (7-acétyl-1,1,3,4,4,6-hexaméthyltétraline ; origine : PFW, Hollande), on a certes obtenu à chaque fois une composition à caractère musqué, mais dont la note était totalement dépourvue du caractère de type musc-nitré, à savoir, doux, poudré et presque vanillé que l'on obtient soit avec la musc cétone, soit avec la 3-méthyl-cyclopentadéc-5-én-1-one selon l'invention.

### Exemple 2

### Préparation d'un détergent en poudre parfumé

On a ajouté à un détergent en poudre non parfumé 0,1% de chacun des composés suivants pour obtenir 4 échantillons de détergent parfumé :

| Composé | Echantillon |
|---|---|
| (Z)-3-méthyl-cyclopentadéc-5-én-1-one | A |
| (E)-3-méthyl-cyclopentadéc-5-én-1-one | B |
| (Z)-3-méthyl-cyclopentadéc-4-én-1-one | C |
| (E)-3-méthyl-cyclopentadéc-4-én-1-one | D |

On a ensuite lavé séparément 4 lots standard de textiles avec les échantillons A à D. Après le lavage, les quatre lots de textiles ont été évalués à l'aveugle par un panel d'experts parfumeurs. De l'avis de ces derniers, le linge lavé avec les échantillons C et D ne dégageait qu'une odeur musquée de type classique et, par ailleurs, très faible, alors que le linge lavé avec les échantillons A et B développait une fragrance à la fois florale et de type musc-nitré, très intense et de longue durée.

### Exemples 3-12

On a procédé au parfumage des articles mentionnés ci-après dans les concentrations indiquées, par adjonction de 3-méthyl-cyclopentadéc-5-én-1-one aux bases appropriées non parfumées :

| | Article | Conc. (% en poids) | Odeur/Aspect [25°C] | Odeur/aspect [40°C] |
|---|---|---|---|---|
| 3. | Eau de toilette alcoolique (alcool 95°) | 5.0 | S/N | S/N |
| 4. | Crème huile/eau | 0.4 | S/N | S/N |
| 5. | Crème eau/huile | 0.4 | S/N | S/N |
| 6. | Shampoing | 0.5 | S/N | S/N |
| 7. | Désodorisant (spray) | 1.3 | S/N | S/N |
| 8. | Spray laque | 0.2 | S/N | S/N |
| 9. | Savon (suif + huile de coco) | 0.5 | S/N | S/N |
| 10. | Talc | 0.5 | S/N | A/N |
| 11. | Détergent en poudre | 0.2 | S/N | S/N |
| 12. | Antiperspirant roll-on | 0.5 | S/N | S/N |
| Clé des abréviations : S = stable N = normal A = acceptable | | | | |

Les essais de parfumage et stabilité résumés dans le tableau ci-dessus ont montré que la 3-méthyl-cyclopentadéc-5-én-1-one convient parfaitement au parfumage d'articles de consommation variés et peut trouver de ce fait un emploi étendu en parfumerie. Il a été observé qu'elle couvrait efficacement l'odeur de la base, là où il y avait lieu, et qu'elle impartissait à ces produits une odeur musquée, douce et florale très agréable.

## Revendications

1. Utilisation de la 3-méthyl-cyclopentadéc-5-èn-1-one à titre d'ingrédient parfumant, dans la préparation de compositions parfumantes et articles parfumés, pour impartir à ces compositions et articles une note odorante de type musc-nitré.

2. Utilisation selon la revendication 1, caractérisée en ce que la 3-méthyl-cyclopentadéc-5-èn-1-one possède la configuration cis.

3. Utilisation selon la revendication 1, caractérisée en ce que la 3-méthyl-cyclopentadéc-5-èn-1-one possède la configuration trans.

4. Composition parfumante ou article parfumé résultant de l'utilisation selon les revendications 1, 2 ou 3.

5. Composition contenant de la 3-méthyl-cyclopentadéc-5-èn-1-one et de la 3-méthyl-cyclopentadéc-4-èn-1-one, et dont la proportion pondérale de la 1-méthyl-cyc1opentadéc-5-èn-1-one est supérieure ou égale à 70%.

6. Composition selon la revendication 5, caractérisée en ce qu'au moins 30% de la quantité de 3-méthyl-cyclopentadéc-5-èn-1-one dans la composition est constitué par l'isomère cis de cette énone.

7. Utilisation d'une composition selon les revendications 5 ou 6 à titre d'ingrédient parfumant, pour la préparation de compositions parfumantes ou articles parfumés.

8. Composition parfumante ou article parfumé résultant de l'utilisation de la revendication 7.

9. Article parfumé selon la revendication 4 ou 8, sous forme d'un parfum ou d'une eau de cologne, d'un savon, d'un gel de douche ou bain, d'un shampoing ou autre produit d'hygiène capillaire, d'une préparation cosmétique, d'un désodorisant corporel ou d'air ambiant, d'un détergent ou adoucissant textile ou d'un produit d'entretien.

## Patentansprüche

1. Verwendung von 3-Methyl-cyclopentadec-5-en-1-on als Riechstoffbestandteil für die Herstellung von Riechstoffkompositionen und parfümierten Artikeln, um diesen Kompositionen und Artikeln eine Duftnote vom Nitro-moschus Typus zu verleihen.

2. Verwendung gemäß Patentanspruch 1, dadurch gekennzeichnet, daß das 3-Methyl-cyclopentadec-5-en-1-on die cis-Konfiguration besitzt.

3. Verwendung gemäß Patentanspruch 1, dadurch gekennzeichnet, daß das 3-Methyl-cyclopentadec-5-en-1-on die trans-Konfiguration besitzt.

4. Riechstoffkomposition oder parfümierter Artikel resultierend aus der Verwendung gemäß den Patentansprüchen 1, 2 oder 3.

5. Komposition enthaltend das 3-Methyl-cyclopentadec-5-en-1-on und das 3-Methyl-cyclopentadec-4-en-1-on, worin der Gewichtsanteil des 3-Methyl-cyclopentadec-5-en-1-on gleich oder höher als 70% ist.

6. Komposition gemäß Patentanspruch 5, dadurch gekennzeichnet, daß mindestens 30% der Menge des in der Komposition anwesenden 3-Methyl-cyclopentadec-5-en-1-ons aus dem cis-Isomeren dieses Enons besteht.

7. Verwendung einer Komposition gemäß den Patentansprüchen 5 oder 6 als Riechstoffbestandteil für die Herstellung von Riechstoffkompositionen oder parfümierten Artikeln.

8. Riechstoffkomposition oder parfümierter Artikel resultierend aus der Verwendung des Patentanspruches 7.

9. Parfümierter Artikel gemäß den Patentansprüchen 4 oder 8 in Form eines Parfüms, eines Kölnisch-Wassers, einer Seife, eines Dusch- oder Badegels, eines Shampoos oder eines anderen Haarpflegemittels, eines kosmetischen Erzeugnisses, eines Körperdesodorans oder Raumluftverbesserers, eines Detergens oder Textilweichspülers oder eines Haushaltsproduktes.

## Claims

1. Use of 3-methyl-5-cyclopentadecen-1-one as a perfuming ingredient, in the preparation of perfuming compositions and perfumed articles, to impart to said compositions and articles a nitromusk type odor note.

2. Use according to claim 1, characterized in that 3-methyl-5-cyclopentadecen-1-one possesses a cis-configuration.

3. Use according to claim 1, characterized in that 3-methyl-5-cyclopentadecen-1-one possesses a trans configuration.

4. Perfuming composition or perfumed article resulting from the use according to any one of claims 1, 2 or 3.

5. Composition containing 3-methyl-5-cyclopentadecen-1-one and 3-methyl-4-cyclopentadecen-1-one, and wherein the weight proportion of 3-methyl-5-cydopentadecen-1-one is 70% or higher.

6. Composition according to claim 5, characterized in that at least 30% of the amount of 3-methyl-5-cyclopentadecen-1-one in the composition consists of the cis isomer of this enone.

7. Use of a composition according to claims 5 or 6 as a perfuming ingredient for the preparation of perfuming compositions or perfumed articles.

8. Perfuming composition or perfumed article resulting from the use according to claim 7.

9. Perfumed article according to claim 4 or 8, in the form of a perfume, a Cologne, a soap, a bath or shower gel, a shampoo or other hair-care product, a cosmetic preparation, a body or air deodorant, a detergent or a fabric softener, or a household product.
